# EUROPEAN PATENT APPLICATION

(11) **EP 1 970 054 A2**
(43) Date of publication of application: **17.09.2008**
(21) Application number: 08152781.4
(22) Date of filing: 14.03.2008
(51) Int. Cl.: A61K 9/20, A61K 31/4365, A61K 47/44

(54) **Clopidogrel tablets**

(30) Priority: 14.03.2007 IN DE05482007
(71) Applicant: Ranbaxy Laboratories Limited, Haryana 122001, Delhi (IN)
(72) Inventor: Gahoi, Sachin, 243406, Uttar Pradesh (IN); Pai, Raveendra, Gurgaon 122015, Haryana (IN); Singla, Ajay Kumar, 160020, Chandigarh (IN); Rafi, Khalid, 110070, Delhi (IN)
(74) Representative: Cronin, Brian Harold John

(57) **Abstract**

The present invention relates to clopidogrel tablets and process of preparation thereof, wherein the tablets have a reduced tendency of sticking to the punch and/or die surfaces of a conventional tablet compression machine.

## Description

### Field of the Invention

The present invention relates to a tablet of a salt of clopidogrel and process of preparation thereof, wherein the tablet has a reduced tendency of sticking to the punch and/or die surfaces of a conventional tablet compression machine.

### Background of the Invention

Clopidogrel is a platelet aggregation inhibitor which acts by direct inhibition of adenosine diphosphate (ADP) binding to its receptor and of the subsequent ADP-mediated activation of the glycoprotein GP IIb/IIIa complex. Clopidogrel is commercialized in its hydrogensulphate salt form (equivalent to 75mg clopidogrel) as the proprietary PLAVIX® tablets by Sanofi-Aventis Limited, France. It is indicated for use in reduction of recent myocardial infarction, recent stroke or established peripheral arterial disease and acute coronary syndrome. The platelet inhibiting activity of clopidogrel makes it an effective drug for reducing the incidence of ischaemic strokes, heart attacks or diseases like atherosclerosis. U.S. Patent No. 5, 576, 328 describes a method of preventing the occurrence of a secondary ischaemic event by the administration of clopidogrel. Chemically it is designated as methyl (+)-*S*-α-(2-chlorophenyl)-6, 7 - dihydrothieno [3, 2-c] pyridine-5(4*H*)-acetate. It has the following structure:

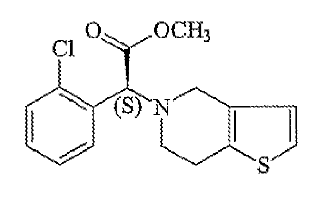

Clopidogrel in its dextrorotatory form is described specifically in U.S. Patent No. 4,847,265. This patent specifically discloses the hydrogensulphate, hydrochloride, hydrobromide and taurocholate salts of clopidogrel. Different polymorphic forms of clopidogrel salts are described in the prior art, for example WO-9965915 discusses a polymorphic form of the clopidogrel hydrogensulphate salt named as Form II and PCT applications WO 2006/034451, WO 2005/026174, WO 2005/068471, WO 2003066637, WO 2005117866 report other polymorphic forms of clopidogrel hydrobromide and clopidogrel hydrochloride.

It is known to one skilled in the art that formulating clopidogrel salts into suitable tablet dosage forms is rather problematic from the manufacturing perspective. The principle reason for such difficulty is attributed to the extreme hygroscopic nature of these salts. Consequently severe adhesion of tablet formulations containing clopidogrel salts to the surfaces of the punches and/or the dies of the conventional tablet compression machines takes place. This causes picking, sticking or other kind of surface irregularities leading to formation of a poor quality finished product. The use of an appropriate lubricant with anti-adherent property could solve the problem of adhesion. However, choice of excipients for manufacturing of clopidogrel tablets is also very critical, because clopidogrel being a thieno [3, 2-c] derivative exhibits rapid degradation when co-processed with certain excipients, namely, alkaline metal salts such as magnesium stearate and sodium stearyl fumarate. Furthermore, certain excipients enhance the inherent stickiness of the clopidogrel salts. Hence, selection of the pharmaceutically acceptable excipients which are compatible with clopidogrel hydrobromide and also reduce its hygroscopicity is critical.

The marketed clopidogrel bisulphate tablets, PLAVIX® comprises of mannitol; microcrystalline cellulose; low substituted hydroxypropyl cellulose; and polyethylene glycol 6000 and hydrogenated castor oil as lubricants [Source: Summary of Product Characteristics, Electronic Medicines Compendium]. Formulations of clopidogrel bisulphate salts are known from WO 2004/098593; EP-B-1310245; WO 2005070464; and WO 2007008045. Clopidogrel hydrobromide and clopidogrel hydrochloride formulations are disclosed in WO-2005/048992 which suggests coating of clopidogrel particles with polyvinyl acetate or a polyvinyl alcohol to overcome hygroscopic nature of clopidogrel. It also suggests a process of preparing stable clopidogrel compositions by dry granulation process using anhydrous excipients. The excipients of choice include mannitol, microcrystalline cellulose, polyethylene glycol 6000 and hydrogenated vegetable oil. However, experiments conducted at our end with the above mentioned excipients resulted in tablets with sticking properties.

Hence, there arises a need to formulate tablets of clopidogrel salts, for example, clopidogrel hydrobromide and clopidogrel hydrochloride, which are free of sticking problem and consequently solve the inherent manufacturing problems related to clopidogrel salts.

### Summary of the Invention

One general aspect, relates to a tablet of a clopidogrel salt comprising a therapeutically effective amount of a clopidogrel salt; lactose; stearic acid; and one or more of other pharmaceutically acceptable excipients; wherein clopidogrel salt is hydrobromide or hydrochloride.

Another aspect, relates to a tablet of a clopidogrel salt comprising a therapeutically effective amount of a clopidogrel salt; lactose and microcrystalline cellulose; stearic acid; and one or more of other pharmaceutically acceptable excipients wherein clopidogrel salt is hydrobromide or hydrochloride.

Yet another aspect, relates to a tablet of a clopidogrel salt comprising a therapeutically effective amount of a clopidogrel salt; lactose; stearic acid and hydrogenated castor oil; and one or more of other pharmaceutically acceptable excipients; wherein clopidogrel salt is hydrobromide or hydrochloride.

Another aspect, relates to a tablet of a clopidogrel salt comprising a therapeutically effective amount of a clopidogrel salt; lactose and microcrystalline cellulose; stearic acid and hydrogenated castor oil; and one or more of other pharmaceutically acceptable excipients; wherein clopidogrel salt is hydrobromide or hydrochloride.

Another aspect, relates to a tablet of a clopidogrel salt comprising a therapeutically effective amount of a clopidogrel salt, prepared by process comprising the steps of
(a) blending the clopidogrel salt with the one or more of the diluents and other pharmaceutically acceptable excipients;
(b) mixing one or more of the lubricants with the blend of step (a); and
(c) compressing the final blend of step (b) into tablet;
wherein the clopidogrel salt is hydrobromide or hydrochloride.

A further aspect, relates to a tablet of a clopidogrel salt comprising a therapeutically effective amount of a clopidogrel salt, prepared by process comprising the steps of
(a) blending the clopidogrel salt with lactose and other pharmaceutically acceptable excipients;
(b) mixing stearic acid with the blend of step (a); and
(c) compressing the final blend of step (b) into tablet;
wherein the clopidogrel salt is hydrobromide or hydrochloride.

Another aspect, relates to a tablet of a clopidogrel salt comprising a therapeutically effective amount of a clopidogrel salt, prepared by process comprising the steps of
(a) blending the clopidogrel salt with lactose and microcrystalline cellulose and optionally other pharmaceutically acceptable excipients;
(b) mixing stearic acid with the blend of step (a); and
(c) compressing the final blend of step (b) into tablet;
wherein the clopidogrel salt is hydrobromide or hydrochloride.

Aanother aspect, relates to a tablet of a clopidogrel salt comprising a therapeutically effective amount of a clopidogrel salt, prepared by process comprising the steps of
(a) blending the clopidogrel salt with lactose and other pharmaceutically acceptable excipients;
(b) mixing stearic acid and hydrogenated castor oil with the blend of step (a); and
(c) compressing the final blend of step (b) into tablet;
wherein the clopidogrel salt is hydrobromide or hydrochloride.

Yet another aspect, relates to a tablet of a clopidogrel salt comprising a therapeutically effective amount of a clopidogrel salt, prepared by process comprising the steps of
(a) blending the clopidogrel salt with lactose and microcrystalline cellulose and optionally other pharmaceutically acceptable excipients;
(b) mixing stearic acid and hydrogenated castor oil with the blend of step (a); and
(c) compressing the final blend of step (b) into tablet;
wherein the clopidogrel salt is hydrobromide or hydrochloride.

### Detailed Description of the Invention

The present invention relates to tablets of a salt of clopidogrel, for example, clopidogrel hydrobromide and clopidogrel hydrochloride, which tablets are free of sticking problems and comprise of a therapeutically effective amount of clopidogrel hydrobromide or clopidogrel hydrochloride; one or more of suitable diluents; one or more of suitable lubricants; and optionally other pharmaceutically acceptable excipients, wherein the said diluents and lubricants help in counteracting the inherent hygroscopicity of clopidogrel salts. It also relates to a process of preparation of such tablets.

Clopidogrel salts as described as herein include hydrobromide and hydrochloride salts and their pharmaceutically acceptable solvates, enantiomers, diastereomers and polymorphs thereof. Clopidogrel salts as described herein are used in a therapeutically effective amount in the tablets. The term "therapeutically effective amount" intends to describe a dose of a clopidogrel salt, with respect to the free base, that is effective for the treatment or prophylaxis of a disease related to inhibition of platelet aggregation activity. The dose may range from about 1mg to about 300mg of clopidogrel, preferably in an amount from about 10mg to about 125mg, more preferably in an amount from about 50mg to about 100mg. The recommended dose of PLAVIX® may be considered as a standard dose.

The choice of diluent is very important, as the inherent adhering tendency of a clopidogrel salt is enhanced by the use of certain diluents. The use of mannitol was found to enhance the stickiness of the clopidogrel tablets. The use of mannitol in combination with microcrystalline cellulose did not reduce the sticking tendency. Changing the amount of either mannitol or microcrystalline cellulose also did not alleviate the sticking problem. Replacing microcrystalline cellulose with lactose in the microcrystalline cellulose/mannitol combination did not yield any favourable results. Sticking problem persisted even when the amount of either lactose or mannitol were varied. However, the use of lactose alone significantly reduced sticking tendency of tablets. The combination of lactose and microcrystalline cellulose also showed favourable results. The combination product of lactose and microcrystalline cellulose, like those available from Meggle-Pharma under the brand name Cellactose® may also be used. These are co-spray dried mixtures of lactose and microcrystalline cellulose. Lactose may be anhydrous lactose and lactose monohydrate. The diluent(s) may comprise from about 40% to about 85%, and preferably from about 50% to about 75% by weight of the tablet.

A lubricant facilitates tablet manufacture by reducing friction in the tablet die during compression and ejection. It also prevents the tablet from sticking to the surface of tablet punch and die wall. Hence, a suitable lubricant with optimum anti-adherent property may be used to counter the problems encountered while processing clopidogrel tablets. Not all lubricants were found suitable as shown by our experiments. Glyceryl behenate, polyethylene glycol and hydrogenated castor oil did not remove sticking tendency of the tablets. Use of a combination of polyethylene glycol and hydrogenated castor oil also did not reduce sticking tendency. However, stearic acid showed favourable results. The combination of stearic acid with hydrogenated castor oil was also found to be suitable. The preferred lubricant is stearic acid and combination of stearic acid and hydrogenated castor oil. The lubricant may comprise from about 1% to about 6% by weight of the tablet.

The tablets may include other pharmaceutically acceptable excipients, without limitation, one or more of suitable disintegrants and glidants.

Disintegrants may be selected from the group consisting of croscarmellose sodium, sodium starch glycolate, crospovidone, low-substituted hydroxypropylcellulose, alginates, carboxymethyl starches, methacrylic acid divinylbenzene copolymer salts and microcrystalline cellulose. The disintegrant(s) comprise about 1% to about 20% by weight of the tablet. In one embodiment, the preferred disintegrant is low-substituted hydroxypropylcellulose.

Glidants may be selected from talc, colloidal silicon dioxide and the like. Suitable glidants may be present in an amount of about 0.1% to about 3% by weight of the tablet. Preferred glidant is colloidal silicon dioxide.

The tablets of clopidogrel salts free of sticking tendency as described herein may be prepared by direct compression. Keeping in view the hygroscopic nature of the clopidogrel salts, it would be advantageous to maintain a relative humidity below 30% while processing such tablets. In one embodiment, the tablets are prepared by direct compression process comprising the steps of
(a) blending clopidogrel salt with the one or more of the diluents and optionally other pharmaceutically acceptable excipients in a suitable blender;
(b) mixing one or more of the lubricants with the blend of step (a);
(c) compressing the final blend of step (b) into tablets.
In another embodiment, the tablets are prepared by the process comprising the steps of
(a) blending clopidogrel salt with lactose and optionally other pharmaceutically acceptable excipients in a suitable blender;
(b) mixing stearic acid with the blend of step (a);
(c) compressing the final blend of step (b) into tablets.
In another embodiment, the tablets are prepared by the process comprising the steps of
(a) blending clopidogrel salt with lactose and microcrystalline cellulose and optionally other pharmaceutically acceptable excipients in a suitable blender;
(b) mixing stearic acid with the blend of step (a);
(c) compressing the final blend of step (b) into tablets.
In another embodiment, the tablets are prepared by the process comprising the steps of
(a) blending clopidogrel salt with lactose, microcrystalline cellulose and optionally other pharmaceutically acceptable excipients in a suitable blender;
(b) mixing stearic acid and hydrogenated castor oil with the blend of step (a);
(c) compressing the final blend of step (b) into tablets.
In another embodiment, the tablets are prepared by the process comprising the steps of
(d) blending clopidogrel salt with lactose and optionally other pharmaceutically acceptable excipients in a suitable blender;
(e) mixing stearic acid and hydrogenated castor oil with the blend of step (a);
(f) compressing the final blend of step (b) into tablets.

The tablets of clopidogrel salts free of sticking and process of preparation thereof described herein is further illustrated by the following examples, but, these should not be construed as limiting the scope of invention.

**Table 1: Compositions of tablets comprising clopidogrel hydrobromide**

| **Ingredients** | **Quantity in mg/tablet** | | | | | | | | | | | |
|---|---|---|---|---|---|---|---|---|---|---|---|---|
| | **Examples** | | | | | | | | | | | |
| | **A** | **B** | **C** | **D** | **E** | **F** | **G** | **H** | **I** | **J** | **K** | **L** |
| Clopidogrel hydrobromide [equivalent to 75mg clopidogrel base] | 93.9 | 93.9 | 93.9 | 93.9 | 93.9 | 93.9 | 93.9 | 93.9 | 93.9 | 93.9 | 93.9 | 93.9 |
| Microcrystalline cellulose | 35 | 225.25 | - | - | - | 35 | 35 | 35 | 35 | 35 | 35 | 35 |
| Mannitol | 190.25 | - | 225.25 | 35 | - | - | - | - | - | - | - | - |
| Lactose monohydrate | - | - | - | - | - | - | - | - | - | - | - | 190.25 |
| Anhydrous lactose | - | - | - | 190.25 | 225.25 | 190.25 | 190.25 | 190.25 | 190.25 | 190.25 | 195.25 | - |
| L-hydroxypropylcellulose | 17.5 | 17.5 | 17.5 | 17.5 | 17.5 | 17.5 | 17.5 | 17.5 | 17.5 | 17.5 | 17.5 | 17.5 |
| Hydrogenated castor oil | 6 | 6 | 6 | 6 | 6 | 6 | 7 | 13 | - | - | - | 6 |
| Stearic acid | 7 | 7 | 7 | 7 | 7 | 7 | - | - | - | - | 8 | 7 |
| Glyceryl behenate | - | - | - | - | - | - | - | - | - | 13 | - | - |
| Polyethylene glycol 6000 | - | - | - | - | - | - | 6 | - | 13 | - | - | - |
| Colloidal silicon dioxide | 0.35 | 0.35 | 0.35 | 0.35 | 0.35 | 0.35 | 0.35 | 0.35 | 0.35 | 0.35 | 0.35 | 0.35 |
| **Total weight** | **350** | **350** | **350** | **350** | **350** | **350** | **350** | **350** | **350** | **350** | **350** | **350** |
| **Presence of stickiness** | Yes | Yes | Yes | Yes | **No** | **No** | Yes | Yes | Yes | Yes | **No** | **No** |

**Table 2: Composition of tablet comprising clopidosrel hydrochloride**

| **Ingredients** | **Quantity in mg/tablet Example M** |
|---|---|
| Clopidogrel hydrochloride [equivalent to 75mg clopidogrel base] | 83.5 |
| Microcrystalline cellulose | 35 |
| Anhydrous lactose | 200.65 |
| L-hydroxypropylcellulose | 17.5 |
| Hydrogenated castor oil | 6 |
| Stearic acid | 7 |
| Colloidal silicon dioxide | 0.35 |
| **Total weight** | **350** |
| **Presence of stickiness** | **No** |

### Procedure:

### Example A

Clopidogrel hydrobromide was mixed with microcrystalline cellulose, mannitol and L-hydroxypropylcellulose and blended with colloidal silicon dioxide. The above blend was further mixed with hydrogenated castor oil and stearic acid to prepare the final blend. The final blend was compressed using conventional tabletting machine. It was observed that severe sticking to the punch and/or die surfaces occurred.

### Example B

Clopidogrel hydrobromide was mixed with microcrystalline cellulose and L-hydroxypropylcellulose and blended with colloidal silicon dioxide. The above blend was further mixed with hydrogenated castor oil and stearic acid to prepare the final blend. The final blend was compressed using conventional tabletting machine. It was observed that severe sticking to the punch and/or die surfaces occurred.

### Example C

Clopidogrel hydrobromide was mixed with mannitol and L-hydroxypropylcellulose and blended with colloidal silicon dioxide. The above blend was further mixed with hydrogenated castor oil and stearic acid to prepare the final blend. The final blend was compressed using conventional tabletting machine. It was observed that severe sticking to the punch and/or die surfaces occurred.

### Example D

Clopidogrel hydrobromide was mixed with mannitol and anhydrous lactose and L-hydroxypropylcellulose and blended with colloidal silicon dioxide. The above blend was further mixed with hydrogenated castor oil and stearic acid to prepare the final blend. The final blend was compressed using conventional tabletting machine. It was observed that severe sticking to the punch and/or die surfaces occurred.

### Example E

Clopidogrel hydrobromide was mixed with anhydrous lactose and L-hydroxypropylcellulose and blended with colloidal silicon dioxide. The above blend was further mixed with hydrogenated castor oil and stearic acid to prepare the final blend. The final blend was compressed using conventional tabletting machine. It was observed that sticking to the punch and/or die surfaces did not occur.

### Example F

Clopidogrel hydrobromide was mixed with microcrystalline cellulose and anhydrous lactose and L-hydroxypropylcellulose and blended with colloidal silicon dioxide. The above blend was further mixed with hydrogenated castor oil and stearic acid to prepare the final blend. The final blend was compressed using conventional tabletting machine. It was observed that sticking to the punch and/or die surfaces did not occur.

### Example G

Clopidogrel hydrobromide mixed with microcrystalline cellulose and anhydrous lactose and L-hydroxypropylcellulose and blended with colloidal silicon dioxide. Hydrogenated castor oil and polyethylene glycol 6000 were blended with the above mixture to prepare the final blend. The final blend was compressed using conventional tabletting machine. It was observed that severe sticking to the punch and/or die surfaces occurred.

### Example H

Clopidogrel hydrobromide mixed with microcrystalline cellulose and anhydrous lactose and L-hydroxypropylcellulose and blended with colloidal silicon dioxide. Hydrogenated castor oil was blended with the above mixture to prepare the final blend. The final blend was compressed using conventional tabletting machine. It was observed that severe sticking to the punch and/or die surfaces occurred.

### Example I

Clopidogrel hydrobromide mixed with microcrystalline cellulose and anhydrous lactose and L-hydroxypropylcellulose and blended with colloidal silicon dioxide. Polyethylene glycol 6000 was blended with the above mixture to prepare the final blend. The final blend was compressed using conventional tabletting machine. It was observed that severe sticking to the punch and/or die surfaces occurred.

### Example J

Clopidogrel hydrobromide mixed with microcrystalline cellulose and anhydrous lactose and L-hydroxypropylcellulose and blended with colloidal silicon dioxide. Glyceryl behenate was blended with the above mixture to prepare the final blend. The final blend was compressed using conventional tabletting machine. It was observed that severe sticking to the punch and/or die surfaces occurred.

### Example K

Clopidogrel hydrobromide mixed with microcrystalline cellulose and anhydrous lactose and L-hydroxypropylcellulose and blended with colloidal silicon dioxide. Stearic acid was blended with the above mixture to prepare the final blend. The final blend was compressed using conventional tabletting machines. It was observed that sticking to the machine punch and/or die surfaces did not occur.

### Example L

Clopidogrel hydrobromide was mixed with lactose monohydrate and L-hydroxypropylcellulose and blended with colloidal silicon dioxide. The above blend was further mixed with hydrogenated castor oil and stearic acid to prepare the final blend. The final blend was compressed using conventional tabletting machine. It was observed that sticking to the punch and/or die surfaces did not occur.

### Example M

Clopidogrel hydrochloride was mixed with microcrystalline cellulose and anhydrous lactose and L-hydroxypropylcellulose and blended with colloidal silicon dioxide. The above blend was further mixed with hydrogenated castor oil and stearic acid to prepare the final blend. The final blend was compressed using conventional tabletting machine. It was observed that sticking to the punch and/or die surfaces did not occur.

## Claims

1. A tablet of a clopidogrel salt comprising a therapeutically effective amount of a clopidogrel salt; lactose; stearic acid; and one or more of other pharmaceutically acceptable excipients; wherein clopidogrel salt is hydrobromide or hydrochloride.

2. The tablet of clopidogrel salt according to claim 1 wherein the amount of lactose is from about 40% to about 85% by weight of the tablet.

3. The tablet of clopidogrel salt according to claim 1 wherein lactose is lactose monohydrate or anhydrous lactose.

4. The tablet of clopidogrel salt according to claim 1 wherein the amount of stearic acid is from about 1% to about 6% by weight of the tablet.

5. The tablet of clopidogrel salt according to claim 1, wherein the tablet further comprises microcrystalline cellulose.

6. The tablet of clopidogrel salt according to claim 5, wherein lactose and microcrystalline cellulose are present as co-spray dried mixture.

7. The tablet of clopidogrel salt according to claim 1 or 5, wherein the tablet further comprises hydrogenated castor oil.

8. A process for the preparation of a tablet of clopidogrel salt according to claim 1 to 7 wherein the tablet is prepared by the process of direct compression.

9. A process for the preparation of a tablet of clopidogrel salt according to Claim 8 wherein the processing is carried out at a relative humidity of below 30%.
